(19) Europäisches Patentamt
European Patent Office
Office européen des brevets

(11) Publication number: **0 145 121 B1**

(12) **EUROPEAN PATENT SPECIFICATION**

(45) Date of publication of patent specification: **14.08.91**

(51) Int. Cl.$^5$: **C07D 471/04**, C07D 495/20, A61K 31/505, //(C07D471/04, 231:00,221:00),(C07D495/20, 331:00,231:00,221:00)

(21) Application number: **84305685.4**

(22) Date of filing: **21.08.84**

(54) **Pyrazoloquinolines, process for their preparation, and pharmaceutical compositions containing them.**

(30) Priority: **26.09.83 US 535519**

(43) Date of publication of application:
**19.06.85 Bulletin 85/25**

(45) Publication of the grant of the patent:
**14.08.91 Bulletin 91/33**

(84) Designated Contracting States:
**BE CH DE FR IT LI LU NL SE**

(56) References cited:
**EP-A- 0 013 787**
**EP-A- 0 023 773**
**GB-A- 2 129 423**
**SU-A- 776 048**
**US-A- 4 230 861**
**US-A- 4 367 231**

**J.Pharm. Exper. Therap. 1983, 324, 206-214**

(73) Proprietor: **ELI LILLY AND COMPANY**
**307, East McCarty Street**
**Indianapolis Indiana 46285(US)**

(72) Inventor: **Schaus, John Mehnert**
**5427 North Delaware Street**
**Indianapolis Indiana 46220(US)**
Inventor: **Huser, Diane Lynn**
**5620 North Broadway**
**Indianapolis Indiana 46220(US)**
Inventor: **Booher, Richard Nolen**
**6886 Hillcrest Court**
**Indianapolis Indiana 46227(US)**

(74) Representative: **Tapping, Kenneth George et al**
**Lilly Industries Limited Patent Department**
**Erl Wood Manor**
**Windlesham Surrey, GU20 6PH(GB)**

**EP 0 145 121 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention concerns novel tautomeric trans-(±)-alkylated-octahydropyrazolo [4,3-f]quinolines, useful as anti-hypertensive agents, a process for their preparation, and novel intermediates.

Tautomeric trans-(±)-5-substituted-7-permissibly-substituted-4,4a,5,6,7,8,8a,9-octahydro-1H(and 2H)-pyrazolo[3,4-g]quinolines (I and II), active prolactin inhibitors and useful in the treatment of Parkinsonism, are described in U.S. Patents 4,198,415, 4,230,861 and 4,367,231.

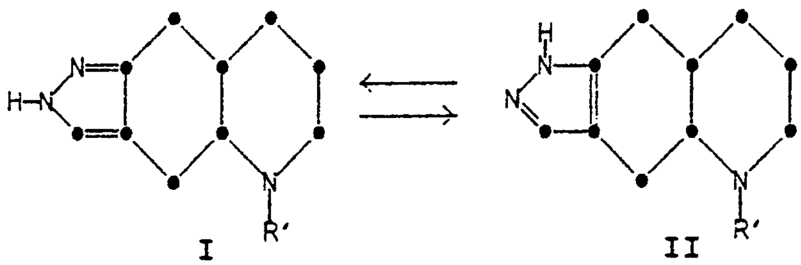

I II

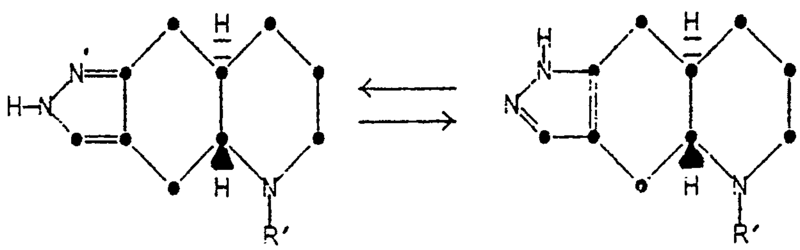

III IV

in which R' is $C_1$-$C_3$ alkyl or allyl.

The hypotensive activity of one of the trans-(-)-stereoisomers, 4aR,8aR-5-n-propyl-4,4a,5,6,7,8,8a,9-octahydro-1H(and 2H)-pyrazolo[3,4-g]quinoline (the tautomeric pair III and IV where R' is n-propyl) is disclosed by Hahn et al., J. Pharm. Exper. Therap., 206 (1983).

The octahydropyrazoloquinolines of the above formula I-IV are prepared according to Reaction Scheme I.

Reaction Scheme I

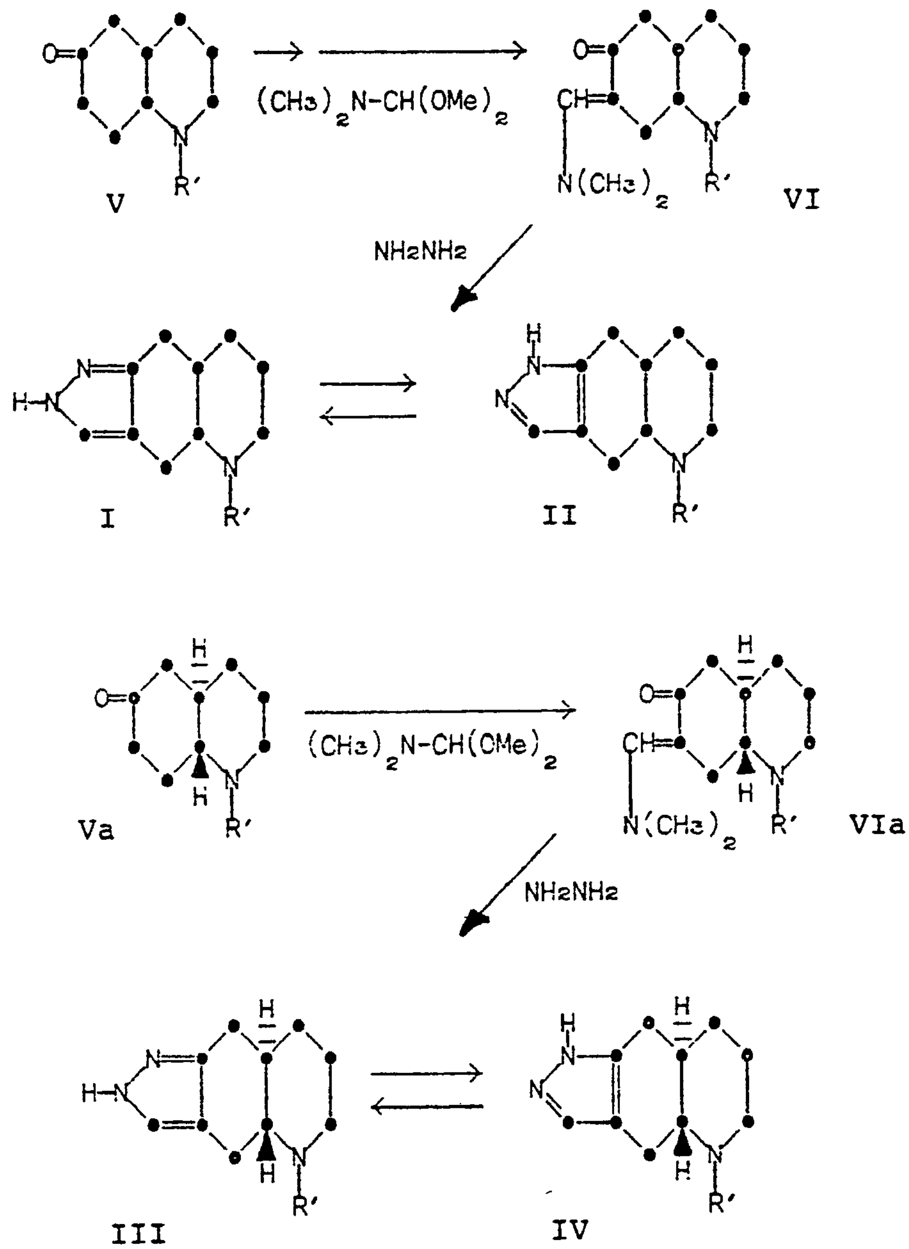


V VI I II Va VIa III IV

wherein R' is $C_1$-$C_3$ alkyl or allyl.

In this scheme, a trans-(±)-1-alkyl-(or allyl)-6-oxodecahydroquinoline (V) is condensed with dimethylformamide dimethylacetal to yield (VI) which, on reaction with hydrazine, yields the tautomeric pair (I) and (II).

British published application 2,129,422 provides a method of resolving (V) to yield the trans-(-)-(4aR,8aR)stereoisomer (Va), or trans-(+)-stereoisomer condensation of which with dimethylformamide dimethylacetal yields (VIa) which, on reaction with hydrazine, yields the tautomeric pair (III) and (IV). The corresponding trans-(+) pair is prepared in an analogous manner from the trans-(+)-(4aS,9aS)stereoisomer.

British published application 2,130,576, provides an alternate intermediate, trans-(±), trans-(+) or trans-(-)-7-formyl-1-alkyl-6-oxodecahydroquinoline which, on reaction with hydrazine, yields the tautomeric pairs (I) and (II) or (III) and (IV), and the trans-(+) pair depending on whether the trans-(±)-racemate, the trans-(+) or the trans-(-)-stereoisomer is used as the starting material.

The bicyclic ketones (V) and (Va) are also used as a starting material to prepare a series of octahydro-2H-pyrrolo[3,4-g]quinolines via the intermediates (VI) and (VIa) which are condensed with a glycinate salt (rather than hydrazine) to yield the desired tricyclic compounds--see U.S. Patent 4,311,844. Compounds of formulae I-IV in which R' is allyl and the corresponding pyrroloquinolines are prepared from products in

which R' is CN or benzyl, by removing the CN or benzyl group and then allylating the resulting secondary amine wherein R' is H with an allyl halide under standard conditions.

The prior art cited above does not provide octahydropyrazolo[3,4-g]quinolines having an alkyl substituent in the 3-position of the pyrazole ring; does not provide methods for their synthesis; does not give a use.

This invention provides tautomeric trans-(±)-alkylated octahydropyrazolo [4,3-f]quinolines of the structures XII and XIII below:

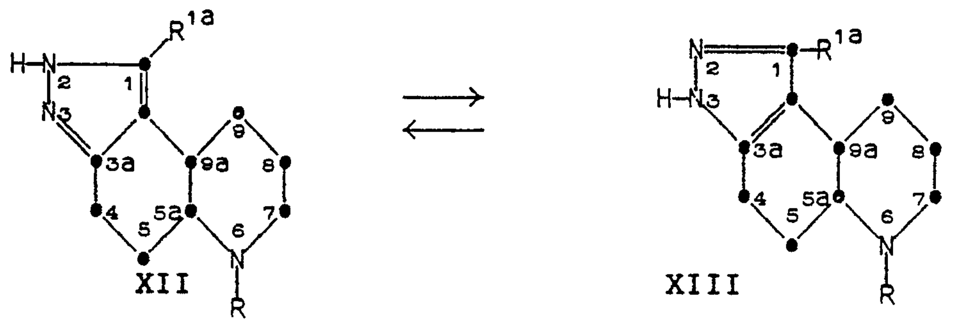

XII XIII

For example, trans-(±)-1,6-dialkyl-4,5,5a,6,7,8,9,9a-octahydro-2H-pyrazolo[4,3-f]-quinoline

For example, trans-(±)-1,6-dialkyl-4,5,5a,6,7,8,9,9a-octahydro-3H-pyrazolo[4,3-f]-quinoline

wherein R is CN, H, $C_1$-$C_3$ alkyl or allyl; $R^{1a}$ is $C_1$-$C_3$ alkyl or H; or a pharmaceutically-acceptable acid addition salt thereof.

Structures represented by (XII) and (XIII) are tautomers; i.e., there is a dynamic equilibrium between each pair of structures shown. When any single member of a tautomeric pair is described, it will be understood that the other tautomer is also described thereby.

The compounds of formulae XII and XIII are prepared by reacting a compound of the formula

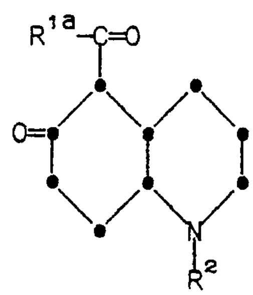

XXIII

wherein $R^{1a}$ is H or $C_1$-$C_3$ alkyl; and $R^2$ is $C_1$-$C_3$ alkyl, allyl, or CN;
with $NH_2NH_2$, or with a salt or hydrate thereof;

optionally resolving the product into its trans-(-)- and trans-(+)-stereoisomers;

optionally salifying the product to form the pharmaceutically-acceptable acid addition salts thereof.

The compounds of formulae XII and XIII can also be prepared by reacting trans-(±)-tautomeric compounds of the formulae

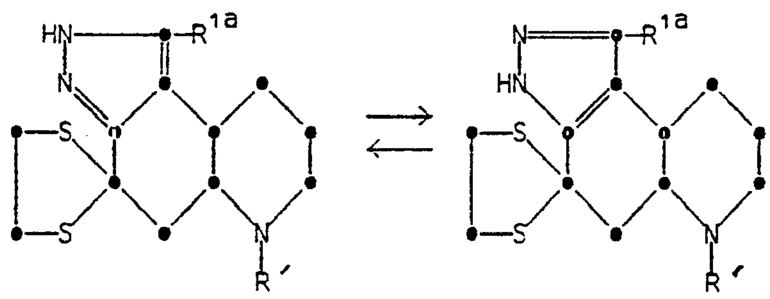

XXIX XXX

where R'is $C_1$-$C_3$ alkyl or allyl; and $R^{1a}$ is H or $C_1$-$C_3$ alkyl;
with Raney Ni;

optionally resolving the product into its trans-(-)- and trans-(+)-stereoisomers;

optionally salifying the product to form the pharmaceutically-acceptable acid addition salts thereof.

In all of the above processes, the resolved trans-(-)- or trans-(+)-starting material can be used. The final product can also be resolved by conventional methods.

Pharmaceutical formulations comprising as active ingredient a tautomeric mixture of compounds of formulae XII and XIII, the resolved stereoisomers thereof, or a pharmaceutically-acceptable acid addition salt thereof, associated with one or more pharmaceutically-acceptable carriers or diluents therefor, are also a part of this invention.

The compounds of formulae XII or XIII, their stereoisomers, or a pharmaceutically-acceptable acid addition salt thereof, can be used for treating hypertension in a mammal by administering to said mammal an effective amount of one or more of the compounds of formulae XII or XIII as defined above. The compounds are therefor useful as hypotensive agents.

Pharmaceutically-acceptable acid addition salts of the compounds of formulae XII or XIII include salts derived from non-toxic inorganic acids such as: hydrochloric acid, nitric acid, phosphoric acid, sulfuric acid, hydrobromic acid, hydriodic acid and phosphorous acid, as well as salts derived from non-toxic organic acids such as aliphatic mono and dicarboxylic acids, phenyl-substituted alkanoic acids, hydroxy alkanoic and alkandioic acids, aromatic acids, aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycollate, malate, tartrate, methanesulfonate, propanesulfonate, naphthalene-1-sulfonate and naphthalene-2-sulfonate.

Compounds represented by the above formulas have two asymmetric centers, at C-5a and C-9a in XII and XIII. Thus, tautomeric pairs, representing four stereoisomers occurring as two racemates, are indicated by XII and XIII. These racemates are designated as the cis-(±) and trans-(±)-racemates. This invention includes only the latter racemates. The trans-(-)-racemate (5aR,9aR-stereoisomer for XII and XIII) is designated by the following formulas.

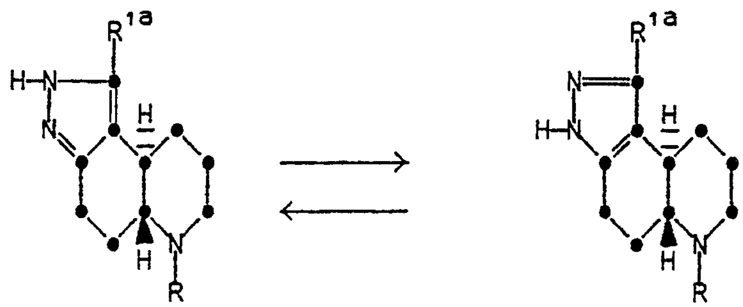

XVI XVII

wherein R and $R^{1a}$ have their previous meanings. The trans-(+) enantiomer would have the opposite configuration for the bridgehead hydrogens; i.e., 5aS,9aS for XVIa and XVIIa:

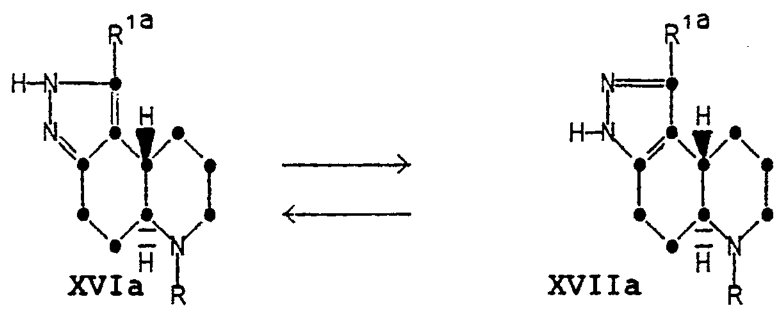

XVIa XVIIa

wherein R and $R^{1a}$ have their previous meanings.

This invention also provides novel pyrazolo[4,3-f]quinolines of the formulas XVIII and XIX:

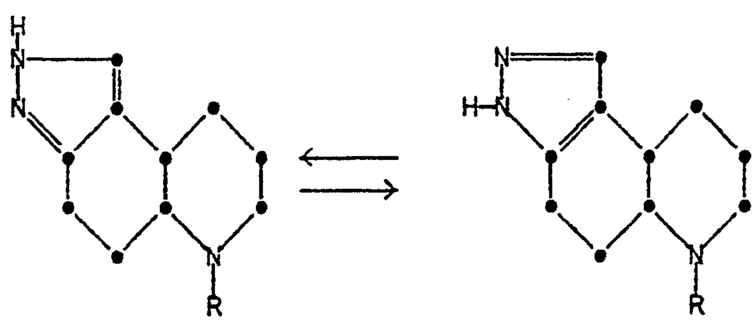

XVIII XIX

wherein R has its previous meaning. (XVIII and XIX correspond to XVI and XVII wherein $R^{1a}$is H). The corresponding trans-(-) tautomers have the formulas XX and XXI below:

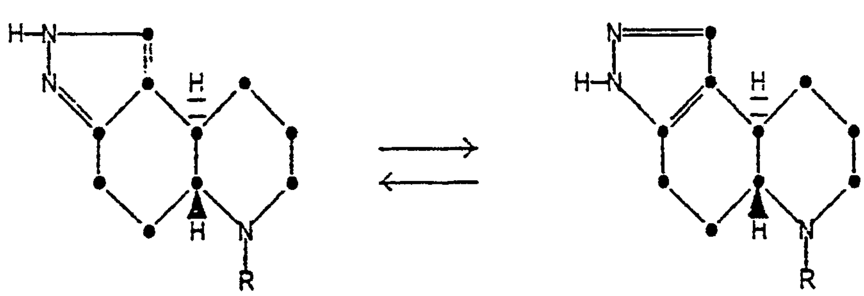

XX XXI

wherein R has its previous meaning. The trans-(+) tautomers have the opposite configuration for the bridgehead hydrogens. Pharmaceutically-acceptable acid addition salts thereof are also contemplated.

Compounds illustrating the scope of formulae X-XIII include the following:

Trans-(-)-1,6-dimethyl-4,5,5a,6,7,8,9,9a-octahydro-2H-(and 3H)-pyrazolo[4,3-f]quinoline tartrate

Trans-(-)-1-methyl-6-n-propyl-4,5,5a,6,7,8,9,9a-octahydro-2H-(and 3H)-pyrazolo[4,3-f]quinoline succinate

Trans-(±)-1-n-propyl-6-methyl-4,5,5a,6,7,8,9,9a-octahydro-2H(and 3H)-pyrazolo[4,3-f]quinoline mesylate

Trans-(+)-1-ethyl-6-allyl-4,5,5a,6,7,8,9,9a-octahydro-2H(and 3H)-pyrazolo[4,3-f]quinoline tosylate.

Tautomeric pairs, of formulae XII-XIII and XVI-XVII, when R is $C_1$-$C_3$ alkyl or allyl and $R^{1a}$ has its previous meaning, are hypotensive agents as are the tautomeric pairs of formulae XVIII-XIX and XX-XXI. The corresponding trans-(+) isomeric pairs of tautomers are also useful drugs. In the same tautomeric pairs when R is H or CN, the compounds are predominantly intermediates, useful in preparing the active hypotensive drugs.

The compounds of formulae XII and XIII are prepared according to Reaction Scheme II below.

## Reaction Scheme II

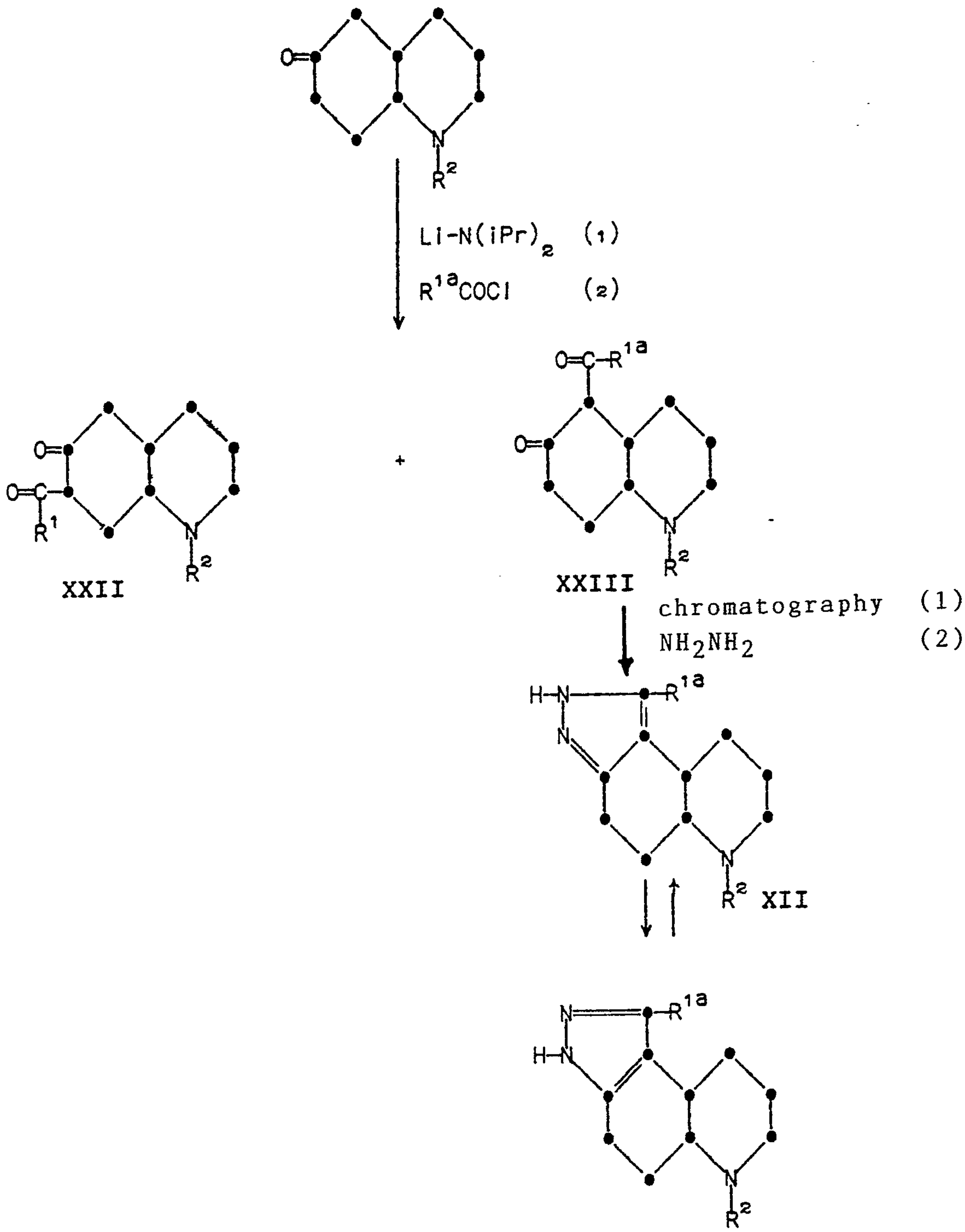


XXII + XXIII

XII

XIII

1 wherein $R^2$ is $C_1$-$C_3$ alkyl, allyl or CN; and $R^{1a}$ is H or $C_1$-$C_3$ alkyl. All formulas represent the trans-(±)-racemate. The trans-(-)-enantiomer as well as the trans-(+)-enantiomer (Va) provided by the method of British published application 2,129,422 undergoes identical reactions to provide intermediates of the structures:

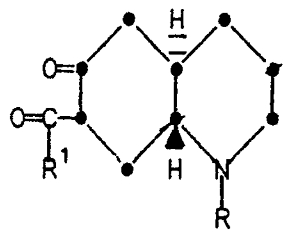

and

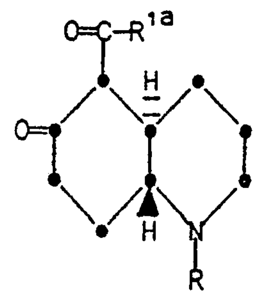

XXIV.    XXV

where R and $R^{1a}$ are defined as above, and the trans-(+) derivatives.

These pairs of products such as XXII and XXIII or XXIV and XXV can be separated into the individual isomers by chromatography. However, we prefer to carry out the cyclization reaction with hydrazine on the mixture of acylated 6-oxodecahydroquinolines to yield a mixture of pyrazoles and then to separate the resulting tautomeric pairs by chromatography.

An alternate method exists for preparing the tautomeric pairs XII and XIII or XVI and XVII. This procedure is outlined in Reaction Scheme III below:

## Reaction Scheme III

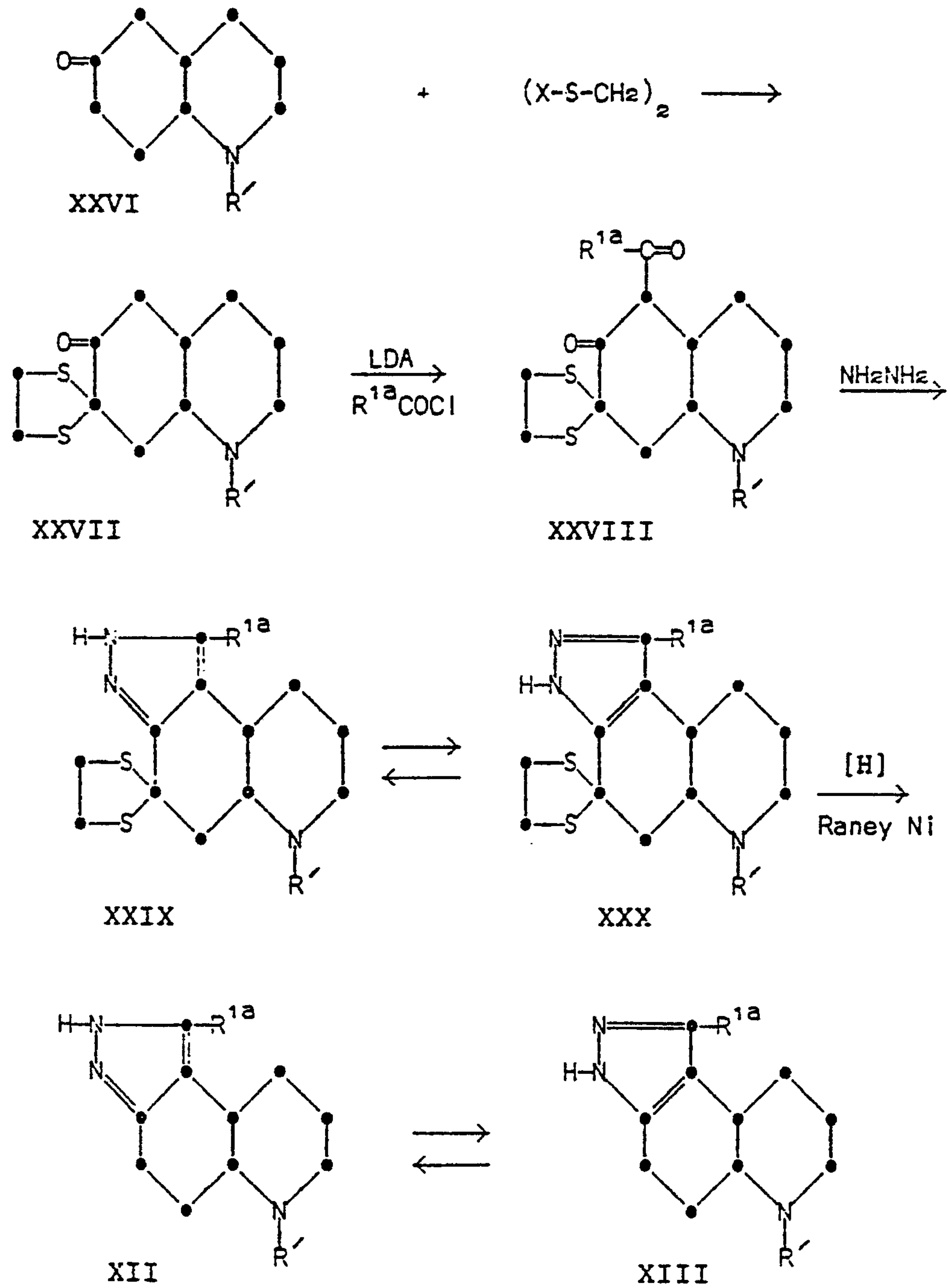


XXVI XXVII XXVIII XXIX XXX XII XIII

wherein R' is $C_1$-$C_3$ alkyl or allyl, X is a leaving group, $R^{1a}$ has its previous meaning.

In Reaction Scheme III, the usual bicycloketone starting material XXVI (V where R is restricted to $C_1$-$C_3$ alkyl), is reacted first with a 1,2-dithioethane derivative X-S-$CH_2$-$CH_2$-S-X wherein X is a leaving group such as tosyl, benzenesulfonyl or mesyl in the presence of pyrrolidine and triethylamine to form a dithioketal at C-7 as a blocking group (XXVII). The C-5 position is then acylated as before with a $C_1$-$C_3$ alkyl halide and lithium diisopropyl amide (LDA, LiN[CH($CH_3$)$_2$]) Cyclization with hydrazine, or salt or hydrate thereof produces the angular octahydropyrazolo[4,3-f]quinoline tautomeric pair (XXIX and XXX) still carrying the thioketal blocking group, which group is readily removed, as by treatment with Raney nickel, to yield compounds of formulae XII and XIII.

Tautomers of structures XVIII-XIX and XX-XXI are prepared by formylating the blocked ketone XVIII at

C-5, employing any of the prior art formylating procedures, for example ethylformate and base. The reactive intermediate XXXIII

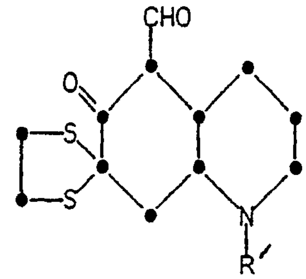

XXXIII

wherein R' is defined as before, is reacted with hydrazine to yield the product XXXIV-XXXV, wherein $R^3$ is defined as before, still containing the blocking group.

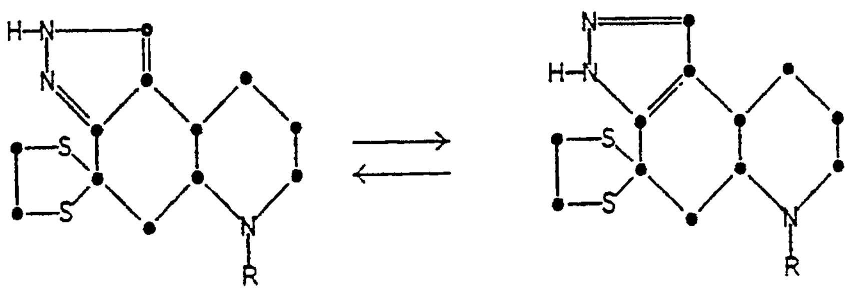

XXXIV XXXV

The blocking group is readily removed, as before, with Raney Ni to yield the pair XVIII-XIX.

The above chemistry is equally adaptable to the preparation of the optically active tautomers XX-XXI by starting with an optically-active starting material XXXVI or its trans-(+) enantiomer.

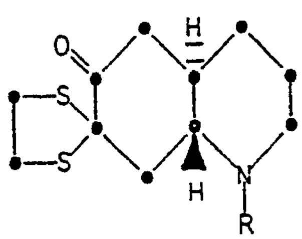

XXXVI

If it is desired to have a 6-allyl group in a final octahydropyrazolo[4,3-f]quinoline, the compound can be prepared by removing the alkyl group $R^3$ from the final tautomeric mixture, as with CNBr, thus forming a compound according to XII-XIII above in which R is CN. Removal of the cyano group yields a derivative wherein R is H, and this secondary amine is readily allylated, as with allyl chloride, to yield the desired derivative.

Those intermediates in which R is H can also be alkylated with a different alkyl group or alkylated with the same alkyl group containing a tagged (radioactive or isotopic) atom to yield useful products.

This invention is further illustrated by the following specific examples.

Preparative Example 1

Preparation of trans-(±)-3-methyl-5-n-propyl-4,4a,5,6,7,8,8a,9-octahydro-1H(and 2H)-pyrazolo[3,4-g]quinoline

A 0.5M lithium diisopropylamide solution was prepared by adding 62.5 ml. of n-butyl lithium (1.6 molar in hexane) to 14 ml. of diisopropylamine in 100 ml. of THF at 0 °C. After the preparation had been completed, the reagent was diluted to 200 ml. giving a 0.5 molar lithium diisopropylamide solution in tetrahydrofuran/hexane solvent mixture. Twenty-two ml. of this solution was placed in a dry 50 ml. round-bottom flask and the solution cooled to about -78 °C. One and ninety-five hundredths grams of trans-(±)-1-n-propyl-6-oxo-decahydroquinoline in 5 ml. of THF were added to the solution and the resulting mixture stirred at about -78 °C. for 30 minutes. Next, 1.06 ml. of acetyl chloride were added and the reaction mixture was allowed to warm to room temperature. The reaction mixture was stirred for two hours at room temperature and was then poured into water. The alkaline aqueous mixture was extracted several times with equal volumes of methylenedichloride. The methylenedichloride extracts were combined and dried and the methylenedichloride removed to yield 2.895 g. of an orange oil comprising trans-(±)-1-n-propyl-6-oxo-7-acetyldecahydroquinoline formed in the above reaction. The product was purified by chromatography over silica using 4% methanol and methylenedichloride plus ammonia as the original eluant. The percent of methanol was raised to 7.5% and fractions containing trans-(±)-1-n-propyl-6-oxo-5-acetyldecahydroquinoline were combined to yield after evaporation of the solvent 728 mg. of a yellow liquid. NMR indicated that these fractions were mixtures of the two products, trans-(±)-1-n-propyl-6-oxo-7-acetyldecahydroquinoline and trans-(±)-1-n-propyl-5-acetyl-6-oxodecahydroquinoline.

The above prepared mixture was dissolved in 20 ml. of methanol to which was added 2 ml. of hydrazine and sufficient 15% aqueous hydrochloric acid to adjust the pH to about 9. The reaction mixture was stirred at room temperature for about one hour and then poured into dilute aqueous sodium hydroxide. The alkali insoluble materials were extracted in methylenedichloride. The methylenedichloride extract was dried and the solvent removed therefrom to yield 959 mg. of a yellow oil which was purified by chromatography over silica using 2:1 THF/hexane solvent mixture containing also aqueous ammonium hydroxide. Fractions shown by TLC to contain trans-(±)-3-methyl-5-n-propyl-4,4a,5,6,7,8,8a,9-octahydro-1H-(and2H)-pyrazolo[3,4-g]quinoline were combined to yield 406 mg. of a light yellow oil. The product had the following physical characteristics:

Ultraviolet spectrum: 220 ($\epsilon$ = 5200).

Infrared spectrum ($CHCl_3$): 3464 $cm^{-1}$; mass spectrum: 233, 204, 125, 124, 96 and 42.

The hydrochloride salt was formed by dissolving the yellow oil in methylenedichloride/ether solvent mixture and saturating with anhydrous gaseous hydrogen chloride. The resulting white solid consisting of trans-(±)-3-methyl-5-n-propyl-4,4a,5,6,7,8,8a,9-octahydro-1H(and 2H)-pyrazolo[3,4-g]quinoline dihydrochloride was separated by filtration. Recrystallization from methanol yielded trans-(±)-3-methyl-5-n-propyl-4,4a,5,6,7,8,8a,9-octahydro-1H(and 2H)-pyrazolo[3,4-g]quinoline dihydrochloride as fine white needles melting about 250 °C.

Analysis Calculated: C, 54.90; H, 8.23; N, 13.72; Cl, 23.15

Found: C, 54.76; H, 8.36; N, 13.72; Cl, 23.37.

Example 2

Preparation of trans-(±)-1-methyl-6-n-propyl-4,5,5a,6,7,8,9,9a-octahydro-2H(and 3H)-pyrazolo[4,3-f]quinoline

The preparation of Example 1 was repeated using 3.9 g. of trans-(±)-1-n-propyl-6-oxo-decahydroquinoline starting material and about 88 ml. of 0.5M lithium diisopropylamine in THF solution. In this preparation, the LDA solution and starting material were combined at 0 °C. after which time the temperature was reduced to -78 °C. and the acetyl chloride added thereto. The reactions were worked up and the mixture products isolated as in Example 1. Five and one tenths grams of this mixture was reacted with 5 ml. of hydrazine in 25 ml. of methanol as in Example 1 to yield a mixture of the [4,3-f] and [3,4-g] isomeric pyrazolo quinolines. Chromatographic separation yielded 0.8 g. of trans-(±)-1-methyl-6-n-propyl-4,5,5a,6,7,8,9,9a-octahydro-2H(and 3H)-pyrazolo[4,3-f]quinoline and 1.8 g. of trans-(±)-3-methyl-5-n-propyl-4,4a,5,6,7,8,8a,9-octahydro-1H(and2H)-pyrazolo[3,4-g]quinoline. Trans-(±)-1-methyl-6-n-propyl-4,5,5a,6,7,8,9,9a-octahydro-2H(and 3H)-pyrazolo[4,3-f]quinoline had the following physical characteristics:

Dihydrochloride salt MP = 249-250 °C.

Analysis Calculated: C, 54.90; H, 8.23; N, 13.72; Cl, 23.15
Found: C, 54.87; H, 8.08; N, 13.52; Cl, 23.36.

Infrared spectrum ($CHCl_3$): 3462, 3210, 2947, 1448; mass spectrum: 233 (molecular ion), 204, 96, 42; proton nmr ($CDCl_3$): 9.80 (bs, 1H); 3.02 (bd, J = 13.5, 1H); 2.30 (s, 3H); 2.06-2.94 (m, 9H); 1.04-1.90 (m, 7H).

Example 3

Preparation of trans-(±)-3-ethyl-5-n-propyl-4,4a,5,6,7,8,8a,9-octahydro-1H(and 2H)-pyrazolo[3,4-g]quinoline and trans-(±)-1-ethyl-6-n-propyl-4,5,5a,6,7,8,9,9a-octahydro-2H(and 3H)-pyrazolo[4,3-f]quinoline

Following the procedure of Example 1, about 44 ml. of a 0.5M lithium diisopropylamine solution were cooled to -78°C. Three and nine tenths grams of trans-(±)-1-n-propyl-6-oxo-decahydroquinoline in THF were added and the reaction stirred at -78°C. for 30 minutes. Two and six tenths ml. of propionyl chloride were then added and the reaction mixture warmed to room temperature and stirred overnight at that temperature. The reaction was worked up and the product isolated with the procedure of Example 1. About 4.2 g. of a mixture containing trans-(±)-1-n-propyl-6-oxo-7-propionyldecahydroquinoline and the 5-propionyl isomer were obtained. The mixture caused to react with hydrazine in methanol without further purification to yield a mixture of the two pyrazole isomers. These isomers were separated by chromatography over silica gel using the same solvent system as previously. Trans-(±)-3-ethyl-5-n-propyl-4,4a,5,6,7,8,8a,9-octahydro-1H(and 2H)-pyrazolo[3,4-g]quinoline thus prepared was converted to both the dihydrobromide and dihydrochloride salts.

Analysis Calculated for the dihydrochloride salt: C, 56.25; H, 8.50; N, 13.12; Cl, 22.14
Found: C, 56.01; H, 8.72; N, 12.85; Cl, 22.03

Melting point = 271-275°C. after recrystallization from methanol/ethyl acetate; infrared spectrum ($CHCl_3$): 3464, 3210, 2951, 1625, 1461;

Mass spectrum: 247 (molecular ion), 218, 125, 96, 41;

proton nmr ($CDCl_3$): 9.92 (bs, 1H); 2.84-3.10 (m, 2H); 2.68-2.84 (m, 2H); 2.44-2.60 (m, 2H); 2.61 (q, J = 8.1, 2H); 2.14-2.32 (m, 4H); 1.91 (bd, J = 13, 1H); 1.62-1.82 (m, 3H); 1.44-1.62 (m, 2H); 1.24 (t, J = 7.6, 3H); 0.91 (t, J = 8.1, 3H).

The dihydrochloride salt of the isomeric material trans-(±)-1-ethyl-6-n-propyl-4,5,5a,6,7,8,9,9a-octahydro-2H(and 3H)pyrazolo[4,3-f]quinoline (separated by the above chromatographic procedure) was converted to the dihydrobromide which melted at 289°C.

Analysis Calculated: C, 44.03; H, 6.65; N, 10.27; Br, 39.05
Found: C, 43.97; H, 6.70; N, 9.99; Br, 39.29.

Infrared spectrum ($CHCl_3$): 3462, 3210, 2952, 1471;

Mass spectrum peaks at 247: (molecular ion), 218, 175, 125, 84; proton nmr ($CDCl_3$): 9.76 (bs, 1H), 3.03 bd, J = 12, 1H); 2.12-2.94 (m, 11H); 1.36-1.90 (m, 5H); 1.22 (t, J = 8.1, 3H); 1.10-1.36 (m, 1H); 0.90 (t, J = 8.1, 3H).

Example 4

Preparation of trans-(±)-6-n-propyl-4,5,5a,6,7,8,9,9a-octahydro-2H(and 3H)-pyrazolo[4,3-f]quinoline

A reaction mixture was prepared from 1 g. of trans-(±)-1-n-propyl-6-oxodecahydroquinoline and 0.355 g. of pyrrolidine (0.43 ml.) in 50 ml. of acetonitrile. A 4A molecular sieve was added and the reaction mixture stirred under a nitrogen atmosphere at reflux temperature for about three hours. The reaction mixture was then cooled to room temperature and 2 ml. of triethylamine were added followed by 2 g. of 1,2-ditosylthioethane. The resulting reaction mixture was heated to reflux temperature overnight with stirring under a nitrogen blanket. After cooling to room temperature, the reaction mixture was filtered and the solvent removed from the filtrate by evaporation in vacuo. Fifty ml. of 0.1N aqueous hydrochloric acid were added and the new mixture warmed to about 100° for about 30 minutes. The reaction mixture was then cooled to room temperature and the acidic layer extracted with methylenedichloride to remove any acid insoluble material. The acidic layer was then made alkaline with an excess of 14N aqueous ammonium hydroxide. The basic mixture was extracted twice with equal volumes of methylenedichloride. The methylenedichloride extracts were combined. The combined extracts were washed with saturated aqueous sodium chloride and were then dried. Removal of the solvent in vacuo yielded 0.8 g. of a solid comprising trans-(±)-1-n-propyl-6-oxo-7-spiro-1',3'-dithiolanodecahydroquinoline formed in the above reaction. The compound was purified by chromatography over Florisil using 1% methanol in chloroform as the eluant. Fractions containing the desired material were combined to yield 0.5 g. of trans-(±)-1-n-propyl-6-oxo-7-spiro-1',3'-dithiolanodecahydroquinoline having the following physical characteristics:

Melting point (after recrystallization from an hexane/ethyl acetate solvent mixture) = 112-113° C.

| | |
|---|---|
| Analysis Calculated: | C, 58.90; H, 8.12; N, 4.91; S, 22.46 |
| Found: | C, 59.12; H, 8.03; N, 4.92; S, 22.36. |

A reaction mixture containing 0.9 g. of trans-(±)-1-n-propyl-6-oxo-7-spiro-1',3'-dithiolanodecahydroquinoline and 2.5 g. of tris(dimethylamino)methane in 30 ml. of toluene were refluxed with stirring for about 4 hours, at which time TLC indicated starting material was no longer present. Volatile constituents were removed in vacuo. The residual oil was dissolved in 50 ml. of methanol and 1 ml. of anhydrous hydrazine was added. This new reaction mixture was stirred overnight at ambient temperature under $N_2$ blanket. Volatile constituents were again removed in vacuo to yield a reddish oil which was purified by chromatography over Florisil using 2% $CH_3OH$ in $CHCl_3$ as the eluant. Fractions containing trans-(±)-4-spiro-1',3'-dithiolano-6-n-propyl-4,5,5a,6,7,8,9,9a-octahydro-2H(and 3H)pyrazolo[4,3-f]quinoline formed in the above reaction were pooled and the solvents removed in vacuo. A methanol solution of the residue was treated with 22 ml. of 0.1N aqueous hydrochloric acid, (one equivalent) thus forming the hydrochloride salt. Recrystallization of the salt from methanol/ethyl acetate gave 0.9 g. of trans-(±)-6-n-propyl-4-spiro-1',3'-dithiolano-4,5,5a,6,7,8,9,9a-octahydro-2H(and 3H)-pyrazolo[4,3-f]quinoline hydrochloride melting above 260° C.

| | |
|---|---|
| Analysis Calculated: | C, 52.08; H, 6.99; N, 12.15; Cl, 10.25; S, 18.54 |
| Found: | C, 52.21; H, 7.09; N, 12.16; Cl, 10.30; S, 18.39. |

A desulfurization mixture was prepared from 0.63 g. of the above compound, 4.8 ml. of Raney Ni and 50 ml. of anhydrous ethanol. The mixture was refluxed with stirring for about 3 hours and was then filtered. Evaporation of the solvent in vacuo yielded an oily solid. Water and aqueous ammonium hydroxide were added and the basic mixture extracted several times with equal volumes of methylenedichloride. These extracts were combined, washed with saturated aqueous sodium chloride and then dried. Chromatography of the residue obtained by evaporation of the solvent over Florisil with 2-4% $CH_3OH$ in $CHCl_3$ as the eluant

yielded 130 mg. of trans-(±)-6-n-propyl-4,5,5a,6,7,8,9,9a-octahydro-2H(and 3H)-pyrazolo[4,3-f]quinoline. Conversion to the dihydrochloride salt (as above) gave 50 mg. of crystalline product melting above 250° C. after recrystallization from methanol/ethyl acetate; molecular ion, 219 by mass spectrograph.

**Analysis Calculated: C, 53.43; H, 7.93; N, 14,38**
**Found: C, 53.54; H, 8.11; N, 14.45.**

Following the above procedure, trans-(±)[or trans-(-)]-1-n-propyl-6-oxo-7-spiro-1',3'-dithiolanodecahydroquinoline can be reacted with an acyl halide and 0.5M lithium diisopropylamide (LDA) reagent in tetrahydrofuran/hexane solvent mixture. The 5-acyl-6-oxo-7-spiro-1',3'-dithiolanodecahydroquinoline thus produced can be cyclized with hydrazine to yield a pyrazole derivative (of Example 1, or 2) containing a spirodithioketal at 7. The thioketal group can then be removed according to the above procedure to give trans-(±)[or trans-(-)]-1-$C_1$-$C_3$ alkyl-6-n-propyl-4,5,5a,6,7,8,9,9a-octahydro-2H(and 3H)-pyrazolo[4,3-f]quinoline.

The (Tosyl-S-$CH_2$)$_2$ is prepared by the procedure of Organic Synthesis, 54, 33, 37, and 39 (1974).

In the above example, there are several preparations of compounds of formula XXII and XXIII when $R^1$ is methyl, ethyl or n-propyl. We have also developed procedures, described in our co-pending application, EP-A-139393, for the prepation of intermediates wherein $R^1$ can also be $C_1$-$C_3$ alkoxy. The process described therein for the preparation of compounds of formula Va initially produces an isomeric mixture which can be separated to produce the intermediates represented by formula XXXVII and XXXVIII below.

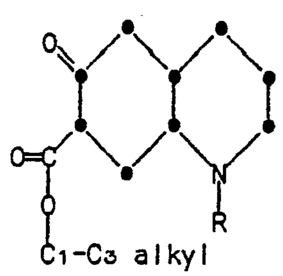

XXXVII

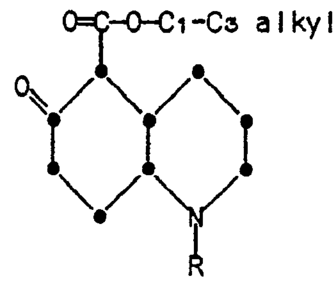

XXXVIII

wherein R has its previous meaning.

While all of the above examples have delineated the preparation of compounds starting with a trans-(±)-bicyclic decahydroquinolinone, it will be apparent to those skilled in the art that identical chemistry would be involved in transforming a trans-(-)-isomer into the corresponding optically active trans-(-) stereoisomeric products and that the trans-(+) tautomers can be prepared in similar fashion.

The tautomeric pairs XII-XIII, and XVIII-XIX, when R is $C_1$-$C_3$ alkyl or allyl and $R^{1a}$ is H, $CH_3$, $C_2H_5$ or n-propyl, reduce the blood pressure of spontaneously hypertensive rats, as illuminated by the following experiment:

Adult male spontaneously hypertensive rats (SHR) (Taconic Farms, Germantown, New York), weighing approximately 300 g. were anesthetized with pentobarbital sodium (60 mg./kg., i.p.). The trachea was cannulated and the SHR respired room air. Pulsatile arterial blood pressure was measured from a cannulated carotid artery using a Statham transducer (P23 ID). Mean arterial blood pressure was calculated as diastolic blood pressure plus 1/3 pulse pressure. Cardiac rate was monitored by a cardiotachometer which was triggered by the systolic pressure pulse. Drug solutions were administered i.v. through a catheter placed in a femoral vein. Arterial blood pressure and cardiac rate were recorded on a multichannel oscillograph (Beckman, Model R511A). Fifteen minutes were allowed to elapse following surgery for equilibration of the preparation. Drugs were administered at a 1 mg./kg. level.

Table 1 which follows gives the results of this test for a series of compounds of formulae XII or XIII In Table 1, column 1 gives the name of the compound, column 2 the change in mean arterial blood pressure, and column 3, the percent change in cardiac rate.

The compounds of formulae XII or XIII, or (+) or (-)isomers thereof, can be administered for therapeutic

purposes in a variety of formulations as illustrated below.

Hard gelatin capsules are prepared using the following ingredients:

| | Quantity (Mg./Capsule) |
|---|---|
| Active compound | 50-100 mg. |
| Starch dried | 200 |
| Magnesium stearate | 10 |

The above ingredients are mixed and filled into hard gelatin capsules.

A tablet formulation is prepared using the ingredients below:

| | Quantity (Mg./Tablet) |
|---|---|
| Active compound | 50-100 mg. |
| Cellulose, microcrystalline | 400 |
| Silicon dioxide, fumed | 10 |
| Stearic acid | 5 |

The components are blended and compressed to form tablets.

Alternatively, tablets each containing 50-100 mg. of active ingredient are made up as follows:

| | |
|---|---|
| Active ingredient | 50-100 mg. |
| Starch | 45 mg. |
| Microcrystalline cellulose | 35 mg. |
| Polyvinylpyrrolidone (as 10% solution in water) | 4 mg. |
| Sodium carboxymethyl starch | 4.5 mg. |
| Magnesium stearate | 0.5 mg. |
| Talc | 1 mg. |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60 °C. and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets.

Capsules each containing 80 mg. of medicament are made as follows:

| | |
|---|---|
| Active ingredient | 50-100 mg. |
| Starch | 59 mg. |
| Microcrystalline cellulose | 59 mg. |
| Magnesium stearate | 2 mg. |

The active ingredient, cellulose, starch and magnesium stearate are blended, passed through a No. 45 mesh U.S. sieve, and filled into hard gelatin capsules.

Suspensions each containing 50-100 mg. of medicament per 5 ml. dose are made as follows:

| | |
|---|---|
| Active ingredient | 50-100 mg. |
| Sodium carboxymethyl cellulose | 50 mg. |
| Syrup | 1.25 ml. |
| Benzoic acid solution | 0.10 ml. |
| Flavor | q.v. |
| Color | q.v. |
| Purified water to | 5 ml. |

The medicament is passed through a No. 45 mesh U.S. sieve and mixed with the sodium carboxymethylcellulose and syrup to form a smooth paste. The benzoic acid solution, flavor and color are diluted with some of the water and added, with stirring. Sufficient water is then added to produce the required volume.

For oral administration, tablets, capsules or suspensions containing from about 50 to about 100 mg. of active drug per dose are given 3-4 times a day, giving a daily dosage of 150 to 400 mgs.

## Claims

1. A trans-(±)-tautomeric mixture of the formulas

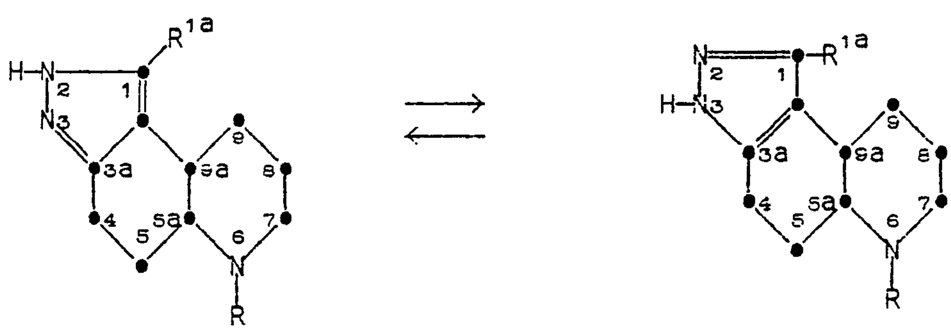

XII XIII

wherein R is H, CN, $C_1$-$C_3$ alkyl or allyl; $R^{1a}$ is H or $C_1$-$C_3$ alkyl, or a pharmaceutically-acceptable acid addition salt thereof.

2. A tautomeric mixture of claim 1 in which R is $C_1$-$C_3$ alkyl or allyl.

3. A tautomeric mixture of claim 1 in which R is H or CN.

4. A dihydrohalide salt of a compound of claim 1 or 2.

5. Any one of the following compounds of claim 1, or a pharmaceutically-acceptable acid addition salt thereof:
trans-(±)-1-methyl-6-n-propyl-4,5,5a,6,7,8,9,9a-octahydro-2H(and 3H)pyrazolo[4,3-f]quinoline
trans-(±)-1-ethyl-6-n-propyl-4,5,5a,6,7,8,9,9a-octahydro-2H(and 3H)pyrazolo[4,3-f]quinoline
trans-(±)-1,6-di-n-propyl-4,5,5a,6,7,8,9,9a-octahydro-2H(and3H)pyrazolo[4,3-f]quinoline.

6. A process for preparing the trans-(±)-tautomeric compounds of formula XII or XIII as defined in claim 1, which comprises reacting a compound of the formula

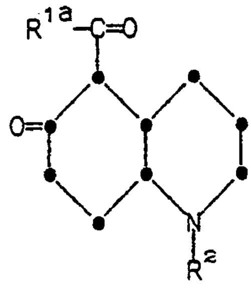

XXIII

wherein $R^{1a}$ is H or $C_1$-$C_3$ alkyl; and $R^2$ is $C_1$-$C_3$ alkyl, allyl or CN;
with $NH_2NH_2$, or with a salt or hydrate thereof;
optionally resolving the product into its trans-(-)- and trans-(+)-stereoisomers;
optionally salifying the product to form the pharmaceutically-acceptable acid addition salts thereof.

**7.** A process for preparing the trans-(±)-tautomeric compounds of the formula XII or XIII as defined in claim 1, which comprises reacting trans-(±)-tautomeric compounds of the formula

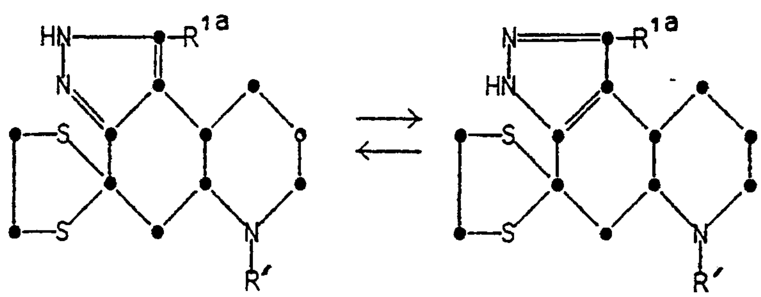

XXIX XXX

where R' is $C_1$-$C_3$ alkyl or allyl; and $R^{1a}$ is H or $C_1$-$C_3$ alkyl;
with Raney Ni;
optionally resolving the product into its trans-(-)- and trans-(+)-stereoisomers;
optionally salifying the product to form the pharmaceutically-acceptable acid addition salts thereof.

**8.** A process of claim 6 or 7 wherein the starting material is the trans-(-)- or trans-(+)-stereoisomer.

**9.** A pharmaceutical formulation comprising as an active ingredient a tautomeric compound of formula XII and XIII as defined in claim 1, a resolved stereoisomer thereof, or a pharmaceutically-acceptable acid addition salt thereof, as claimed in any one of claims 1 to 5, associated with one or more pharmaceutically-acceptable carriers or diluents therefor.

**10.** A compound of formula XII or XIII, a trans-(-)- or trans-(+)-stereoisomer thereof, or a pharmaceutically-acceptable acid addition salt thereof, as claimed in any one of claims 1 to 5 for use as a hypotensive agent.

**11.** A compound of the formula

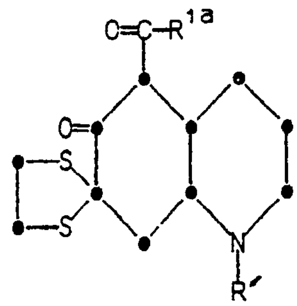

XXVIII

wherein R' is $C_1$-$C_3$ alkyl or allyl; and $R^{1a}$ is H or $C_1$-$C_3$ alkyl; or an acid addition salt thereof.

**12.** A compound of the formula

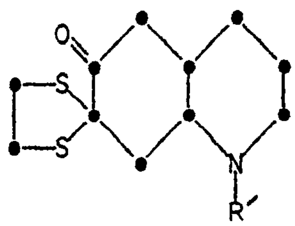

XXVII

wherein R' is $C_1$-$C_3$ alkyl or allyl; or an acid addition salt thereof.

**13.** A trans-(±) tautomeric mixture of the formulas

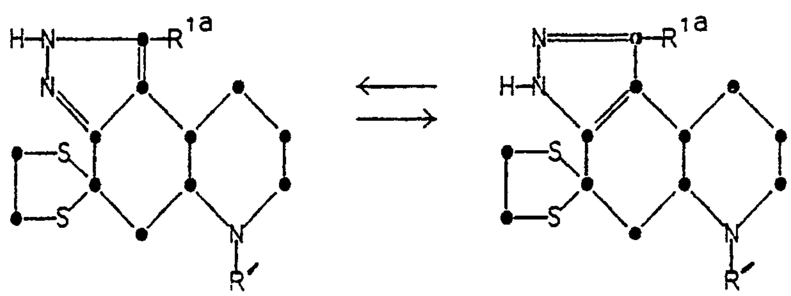

XXIX XXX

wherein R' is $C_1$-$C_3$ alkyl or allyl; and $R^{1a}$ is H or $C_1$-$C_3$ alkyl; or an acid addition salt thereof.

**Revendications**

**1.** Mélange tautomère trans-(±) répondant aux formules

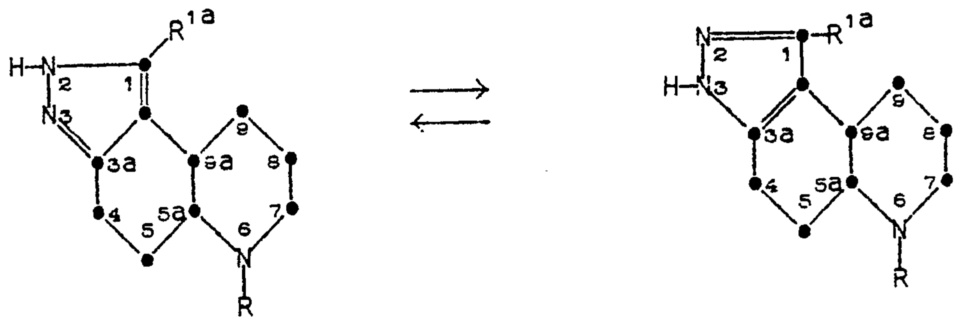

XII XIII

dans lesquelles R représente H, CN, un groupe alkyle en $C_1$-$C_3$ ou un groupe allyle, $R^{1a}$ représente H ou un groupe alkyle en $C_1$-$C_3$ ; ou un de ses sels d'addition d'acide pharmaceutiquement acceptables.

**2.** Mélange tautomère selon la revendication 1, dans lequel R représente un groupe alkyle en $C_1$-$C_3$ ou un groupe allyle.

**3.** Mélange tautomère selon la revendication 1, dans lequel R représente H ou CN.

**4.** Sel dihalogénhydrate d'un composé selon la revendication 1 ou 2.

**5.** L'un quelconque des composés ci-après selon la revendication 1 ou un de ses sels d'addition d'acide pharmaceutiquement acceptables :

trans-(±)-1-méthyl-6-n-propyl-4,5,5a,6,7,8,9,9a-octahydro-2H(et 3H)pyrazolo-[4,3-f]quinoléine ;
trans-(±)-1-éthyl-6-n-propyl-4,5,5a,6,7,8,9,9a-octahydro-2H(et 3H)pyrazolo-[4,3-f]quinoléine ;
trans-(±)-1,6-di-n-propyl-4,5,5a,6,7,8,9,9a-octahydro-2H(et 3H)pyrazolo-[4,3-f]quinoléine.

**6.** Procédé pour la préparation des composés tautomères trans-(±) de formule XII ou XIII, tels que définis dans la revendication 1, consistant à faire réagir un composé de formule

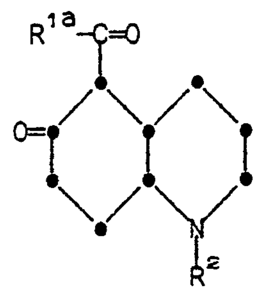

XXIII

dans laquelle $R^{1a}$ représente H ou un groupe alkyle en $C_1$-$C_3$ et $R^2$ représente un groupe alkyle en $C_1$-$C_3$, un groupe allyle ou CN ;

avec $NH_2NH_2$ ou avec un de ses sels ou de ses hydrates;

en dédoublant éventuellement le produit pour obtenir ses stéréo-isomères trans-(-) et trans-(+) ;

en salifiant éventuellement le produit pour obtenir ses sels d'addition d'acide pharmaceutiquement acceptables.

**7.** Procédé pour la préparation des composés tautomères trans-(±) de formule XII ou XIII, tels que définis dans la revendication 1, consistant à faire réagir des composés de formules

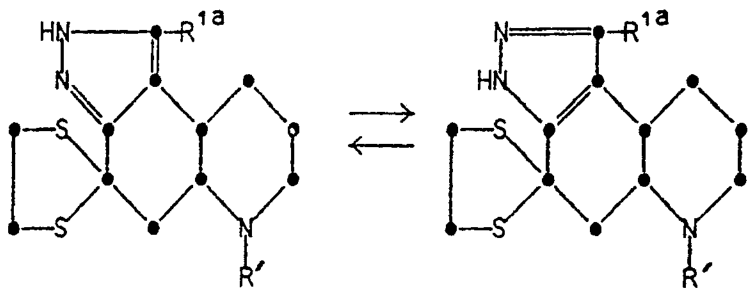

XXIX XXX

où R' représente un groupe alkyle en $C_1$-$C_3$ ou un groupe allyle et $R^{1a}$ représente H ou un groupe alkyle en $C_1$-$C_3$ ;
avec du nickel de Raney ;
en dédoublant éventuellement le produit pour obtenir ses stéréo-isomères trans-(-) et trans-(+) ;
en salifiant éventuellement le produit pour obtenir ses sels d'addition d'acide pharmaceutiquement acceptables.

**8.** Procédé selon la revendication 6 ou 7, dans lequel la matière de départ est le stéréo-isomère trans-(-) ou trans-(+).

**9.** Formulation pharmaceutique comprenant, comme ingrédient actif, un composé tautomère répondant aux formules XII et XIII, tel que défini dans la revendication 1, un de ses stéréo-isomères dédoublé ou encore un de ses sels d'addition d'acide pharmaceutiquement acceptables selon l'une quelconque des revendications 1 à 5, en association avec un ou plusieurs supports ou diluants pharmaceutiquement acceptables pour le composé.

**10.** Composé de formule XII ou XIII, un de ses stéréo-isomères trans-(-) ou trans-(+) ou encore un de ses sels d' addition d'acide pharmaceutiquement acceptables selon l'une quelconque des revendications 1 à 5, destiné à être utilisé comme agent hypotenseur.

**11.** Composé de formule

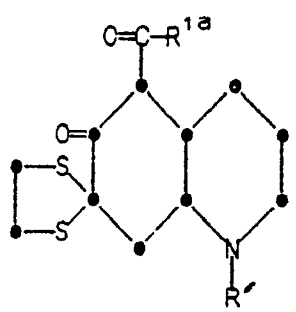

XXVIII

dans laquelle R' représente un groupe alkyle en $C_1$-$C_3$ ou un groupe allyle et $R^{1a}$ représente H ou un groupe alkyle en $C_1$-$C_3$ ou encore un de ses sels d'addition d'acide.

**12.** Composé de formule

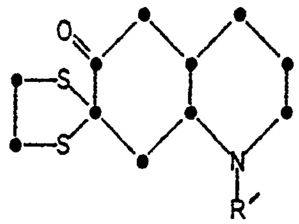

XXVII

dans laquelle R' représente un groupe alkyle en $C_1$-$C_3$ ou un groupe allyle ou encore un de ses sels d'addition d'acide.

**13.** Mélange tautomère trans-(±) répondant aux formules

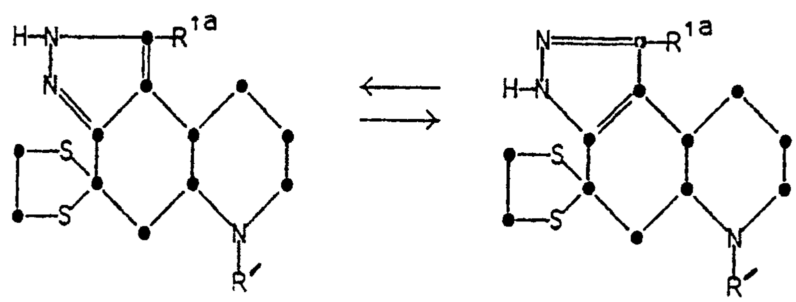

XXIX XXX

où R' représente un groupe alkyle en $C_1$-$C_3$ ou un groupe allyle et $R^{1a}$ représente H ou un groupe alkyle en $C_1$-$C_3$ ou encore un de ses sels d'addition d'acide.

**Patentansprüche**

**1.** Tautomeres Gemisch von trans-(±)-Verbindungen der Formeln

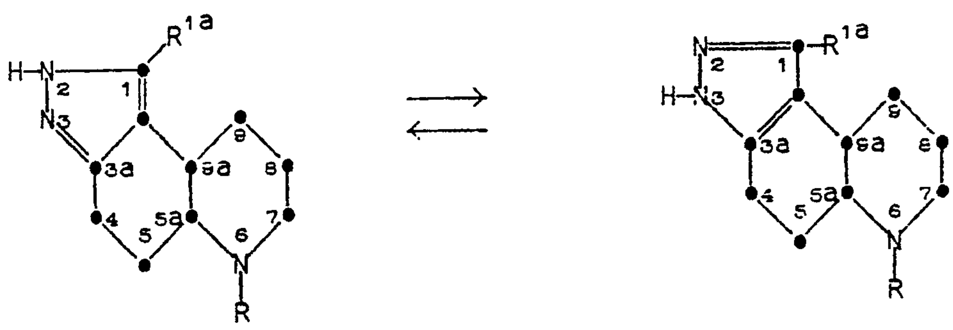

XII XIII

worin R H, CN, $C_1$-$C_3$-Alkyl oder Allyl und $R^{1a}$ H oder $C_1$-$C_3$-Alkyl bedeuten, oder ein pharmazeutisch annehmbares Säureadditionssalz davon.

**2.** Tautomeres Gemisch gemäß Anspruch 1, worin R $C_1$-$C_3$-Alkyl oder Allyl ist.

**3.** Tautomeres Gemisch gemäß Anspruch 1, worin R H oder CN ist.

**4.** Dihydrohalogenid einer Verbindung gemäß Anspruch 1 oder 2.

5. Eine der folgenden Verbindungen gemäß Anspruch 1 oder ein pharmazeutisch annehmbares Säureadditionssalz davon:
trans-(±)-1-Methyl-6-n-propyl-4,5,5a,6,7,8,9,9a-octahydro-2H(und3H)-pyrazolo[4,3-f]chinolin,
trans-(±)-1-Ethyl-6-n-propyl-4,5,5a,6,7,8,9,9a-octahydro-2H(und 3H)-pyrazolo[4,3-f]chinolin,
trans-(±)-1,6-Di-n-propyl-4,5,5a,6,7,8,9,9a-octahydro-2H(und 3H)-pyrazolo[4,3-f]chinolin.

6. Verfahren zur Hestellung der tautomeren trans-(±)-Verbindungen der Formeln XII oder XIII gemäß Anspruch 1, wobei man eine Verbindung der Formel

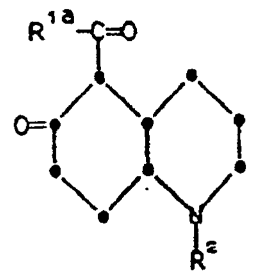

XXIII

worin $R^{1a}$ H oder $C_1$-$C_3$-Alkyl bedeutet und $R^2$ $C_1$-$C_3$-Alkyl, Allyl oder CN bedeutet,
mit $NH_2NH_2$ oder einem Salz oder Hydrat davon umsetzt,
gewünschtenfalls das Produkt der Razemattrennung in die trans-(-)- und trans-(+)-Stereoisomere unterwirft und
gewünschtenfalls aus dem Produkt die pharmazeutisch annehmbaren Säureadditionssalze bildet.

7. Verfahren zur Herstellung der tautomeren trans-(±)-Verbindungen der Formeln XII oder XIII gemäß Anspruch 1, wobei man tautomere trans-(±)-Verbindungen der Formeln

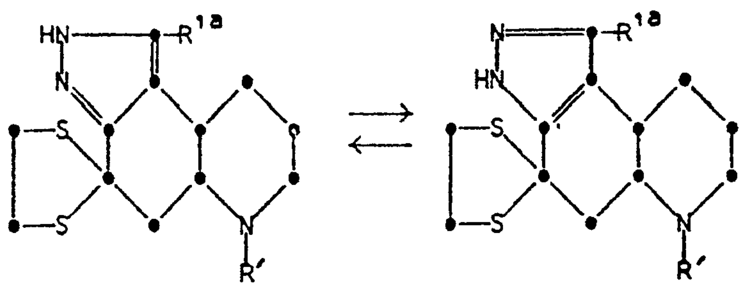

XXIX XXX

worin R' $C_1$-$C_3$-Alkyl oder Allyl und $R^{1a}$ H oder $C_1$-$C_3$-Alkyl bedeuten,
mit Raney-Nickel umsetzt,
gewünschtenfalls das Produkt der Razemattrennung in die trans-(-)- und trans-(+)-Stereoisomere unterwirft und
gewünschtenfalls aus dem Produkt die pharmazeutisch annehmbaren Säureadditionssalze herstellt.

8. Verfahren gemäß Anspruch 6 oder 7, worin das Ausgangsmaterial das trans-(-)- oder trans-(+)-Stereoisomere ist.

9. Pharmazeutische Formulierung, die als Wirkstoff eine tautomere Verbindung der Formel XII oder XIII gemäß Anspruch 1, ein aufgrund von Razemattrennung erhaltenes Stereoisomeres davon oder ein pharmazeutisch annehmbares Säureadditionssalz davon gemäß einem der Ansprüche 1 bis 5 gemeinsam mit einem oder mehreren pharmazeutisch annehmbaren Trägern oder Verdünnungsmitteln dafur enthält.

10. Verbindung gemäß Formel XII oder XIII, ein trans-(-) oder trans-(+)-Stereoisomeres davon oder ein pharmazeutisch annehmbares Säureadditionssalz davon gemäß einem der Ansprüche 1 bis 5 zur

Verwendung als blutdrucksenkendes Mittel.

**11.** Verbindung der Formel

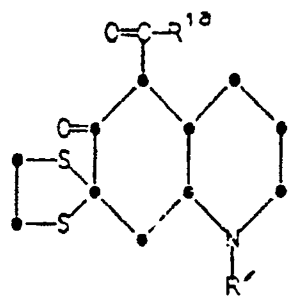

XXVIII

worin R' $C_1$-$C_3$-Alkyl oder Allyl und $R^{1a}$ H oder $C_1$-$C_3$-Alkyl ist, oder ein Säureadditionssalz davon.

**12.** Verbindung der Formel

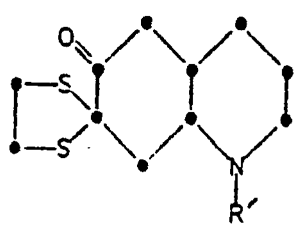

XXVII

worin R' $C_1$-$C_3$-Alkyl oder Allyl ist, oder ein Säureadditionssalz davon.

**13.** Tautomeres Gemisch von trans-(±)-Verbindungen der Formeln

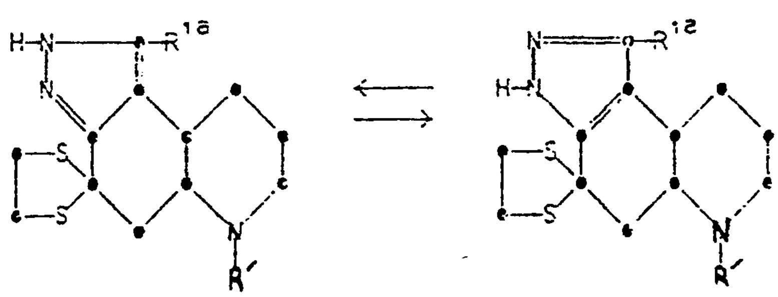

XXIX XXX

worin R' $C_1$-$C_3$-Alkyl oder Allyl und $R^{1a}$ H oder $C_1$-$C_3$-Alkyl ist, oder ein Säureadditionssalz davon.